Europäisches Patentamt

European Patent Office

Office européen des brevets

(11) Publication number: **0 058 574**
**B1**

(12) **EUROPEAN PATENT SPECIFICATION**

(45) Date of publication of patent specification: **29.01.86**

(51) Int. Cl.⁴: **G 02 B 6/00**

(21) Application number: **82300813.1**

(22) Date of filing: **17.02.82**

(54) Method of and apparatus for restoring the light transmittance of an image transmitting optical fiber bundle used in a fiberoptic endoscope.

(30) Priority: **17.02.81 JP 22082/81**
**20.01.82 JP 5907/82**

(43) Date of publication of application:
**25.08.82 Bulletin 82/34**

(45) Publication of the grant of the patent:
**29.01.86 Bulletin 86/05**

(84) Designated Contracting States:
**AT BE CH DE IT LI NL SE**

(56) References cited:
**GB-A-1 041 903**
**US-A-1 662 150**
**US-A-4 053 756**
**US-A-4 157 253**

**SOVIET JOURNAL OF QUANTUM
ELECTRONICS, vol. 9, no. 5, May 1979, pages
636-637, New York, USA E.M. DIANOV et al.:
"Reversible optical bleaching of the induced
absorption in fiber-optic waveguides"**

(73) Proprietor: **Fuji Photo Optical Co., Ltd.**
**1-324 Uetake-cho Omiya-shi**
**Saitama-ken (JP)**

(72) Inventor: **Kojima, Takuo**
**Fuji Photo Optical Co. Ltd. 1-324, Uetake-cho
Omiya-shi Saitama-ken (JP)**
Inventor: **Ishibashi, Kuniaki**
**Fuji Photo Optical Co. Ltd. 1-324 Uetake-cho
Omiya-shi Saitama-ken (JP)**

(74) Representative: **Ayers, Martyn Lewis Stanley
et al**
**J.A. KEMP & CO. 14 South Square Gray's Inn
London, WC1R 5EU (GB)**

(56) References cited:
**OPTICAL ENGINEERING, vol. 18, no. 6,
November/December 1979, pages 552-561,
Bellingham, USA E.J. FRIEBELE: "Optical fiber
waveguides in radiation environments"**

**SOVIET JOURNAL OF QUANTUM
ELECTRONICS, vol. 9, no. 6, June 1979, pages
768-773, New York, USA A.N. GUR'YANOV et
al.: "Radiation-optical stability of low-loss
glass-fiber waveguides"**

Courier Press, Leamington Spa, England.

(56) References cited:
ELECTRONICS LETTERS, vol. 15, no. 4, 15th
February 1979, pages 111-112, Hitchin Herts,
G.B. J.G. TITCHMARSH: "Signal-level
dependence in the recovery rate of irradiated
plastic-clad silica fibre"

APPLIED OPTICS, vol. 20, no. 19, 1st October
1981, pages 3448-3452, New York, USA E.J.
FRIEBELE et al.: "Photobleaching effects in
optical fiber waveguides"

## Description

The present invention relates to a method of and apparatus for restoring the light transmittance of an image transmitting optical fiber bundle which has been reduced by X-ray or γ-ray irradiation and for thus making it acceptable for performing observation or examination.

Fiberoptic endoscopes are widely used for observing or examining inaccessible body cavities which are impossible to directly observe or examine from the outside, and are generally divided into two categories, i.e., medical and industrial ones. Fiberoptic endoscopes for medical use are used to observe or examine cavities of a human body such as the stomach, the duodenum, the colon, and the like, and the others are used to observe or examine the internal parts of machinery such as engines, nuclear reactors and the like. These fiberoptic endoscopes comprise optical fiber bundles, one for transmitting an image of the internal parts to be observed or examined and the other for transmitting illumination light from the outside. Each of said optical fiber bundles comprises an extremely large number of optical fibers with their opposed end portions glued and the major portions between the ends separated so as to be flexible and thus insertable along a tortuous passage of a body.

Upon inserting a fiberoptic endoscope into a human body, a fluoroscopic observation is often taken to accurately determine the inserted position of the top thereof relative to a region within the human body so as to ensure reliably the safety of the person under examination. A certain fiberoptic endoscope for medical use, for instance a duodenum endoscope, can be utilized for the purpose of Endoscopic retrograde cholangiopancreatography (ERCP) examinations wherein a contrast medium is, for a fluoroscopic observation, injected into the pancreatic and bile ducts through a tube which is inserted in a therapeutic instrument guide channel of a fiberoptic endoscope. As described above, fiberoptic endoscopes for medical use are frequently used in combination with a fluoroscopic observation. In consequence of this an optical fiber bundle is frequently exposed to irradiation through a protective rubber tube.

Generally, exposure of optical glasses to X-ray or γ-ray irradiation induces coloration thereof and thus decreases their light transmittance. It has been reported in the literature that the irradiation induced coloration is due to color center. Irradiation interacts with electrons belonging to atoms of glasses to release them. The released electrons impinge upon electrons belonging to other atoms to release them. As a result of this, positive holes having positive charges are created. Although a large number of released electrons are recombined with positive holes, the remaining released electrons are, partially, bound at structural imperfections of optical glass and form color centers. Because these electrons and positives holes at color centers are weakly bound, glass absorbs light with wavelengths longer than the fundamental absorption band of the crystal before irradiation to form another fundamental absorption band in the range of wavelength of visible light.

The irradiation-induced coloration of glass unavoidably occurs in an image transmitting optical fiber bundle having thousands to tens of thousands of glass fibers each of which is composed of a fiber core and fiber cladding and has a diameter of microns in order upon being exposed to irradiation. Therefore, an optical fiber bundle used in a fiberoptic endoscope has many opportunities to be frequently exposed to irradiation and thus increases in absorption of light having wavelengths 400 to 550 nm so as to be colored yellowish brown. The yellowish brown coloration appears on a fiber bundle within a protective tube after irradiation of some Roentgens (R) and develops as the exposure does to the fiber bundle increases. A fiberoptic endoscope having an image transmitting fiber bundle with irradiation-induced coloration unacceptable for observing or examining an image therethrough is returned to a manufacturer to replace the image transmitting fiber bundle. An image transmitting fiber bundle is very expensive, further the replacement of which is extremely complex in operation and hence very costly.

For preventing an optical glass from going yellow, it is known to mix cerium oxide in the composition of the glass, but since a cerium-oxide containing glass has a tinge of yellow in itself, there is a reduction in light transmittance. Optical elements such as optical lenses, prisms and the like, which are thin in the direction of their optical paths, have substantially no effect on observations therethrough even if a decline of light transmittance is caused, while a long optical fiber of diameter 10 to 50 microns and overall length 700 to 1200 nm is influenced considerably, owing to extreme degradation of the light passing therethrough. For this reason, image-transmitting glass fiber bundles conventionally used are made of glass materials without cerium oxide.

An alternative way for preventing irradiation induced coloration of a glass fiber bundle is disclosed in the Japanese Utility Model Publication No. 53-43025, in which an image transmitting fiber bundle is put through a sheath of a concentric helix of metal strip having an irradiation shielding layer of material such as lead, cerium or the like covered. This way has the advantage that the sheath can cause an image transmitting fiber bundle being exposed to less irradiation, while coloration may be induced by irradiation through the spaces in the sheath of the concentric helix of metal strip.

It is known from the literature (see, for example, "Reversible optical bleaching of the induced absorption in Fibre-optic wave-guides" by EM Dianov et al, Soviet Journal of Quantum Electronics, 9(5), May 1979 PP 636—637 and "Optical fiber waveguides in radiation environments" by E. J. Friebele, Optical Engineering Volume 18 No. 6 November—December 1979 PP 552—561) that the increased absorption of a fibre-optic waveguide induced by exposure to ionizing radiation may be reduced by directing intense visible light through the wave guide. To the applicant's knowledge there has been no suggestion that such a technique might be practically applied to reduce the discoloration of the fibre-optic bundle of a fibre-optic endoscope.

The present invention seeks to provide an effective means of restoring the light transmittance of the image transmitting fibre optic bundle of a fibre optic endoscope.

The method according to the present invention is characterised in that the light transmitting bundle being the image transmitting bundle of a fibre optic endoscope, and having an objective lens at its distal end, said image transmitting optical fiber bundle is exposed to visible light radiation by directing said visible light radiation into the bundle through the end from which said image is normally viewed.

A second aspect of the present invention provides a fibre optic endoscope comprising an image transmitting optical fiber bundle having a light transmittance which in use is reduced by irradiation, the bundle having an end from which a transmitted image is normally viewed and an objective lens assembly at its distal end, characterised by the provision, in operative combination with the endoscope, of apparatus for restoring the light transmittance of the bundle, the apparatus comprising:—

a light source which, in use, emits visible light radiation; and

a casing including said light source therein, the casing being adapted to be coupled to said end of the bundle to direct light through the bundle.

The longer the visible light radiating time is, the higher the degree of recovery in light transmittance of an image transmitting fiber bundle is. At a fixed visible light radiation, the higher the radiation density is, the more effective the recovery of light transmittance is. Furthermore short wavelength visible light is particularly effective in restoring light transmittance, and thus a light transmittance closely similar to that before visible light radiation can be obtained in a short time. It can be concluded from these facts that it is desirble to use short wavelength visible light having a high radiation density.

The invention will be further described by way of example with reference to the accompanying drawings in which:—

FIGURE 1 is a schematic illustration of an experimental apparatus;

FIGURE 2 is a graphical representation showing radiation density of the output of a xenon lamp:

FIGURE 3 is a graphical representation showing spectral transmittance factors of a heat absorbing filter and an interference filter;

FIGURE 4 is a graphical representation showing changes in special transmittance factor of an image transmitting fiber bundle with use of an interference filter having a central wavelength of 404 nm;

FIGURE 5 is a graphical representation showing changes in spectral transmittance factor of an image transmitting fiber bundle with use of an interference filter having a central wavelength of 470 nm;

FIGURE 6 is a graphical representation showing changes in a spectral transmittance factor of an image transmiting fiber bundle with use of an interference filter having a central wavelength of 582 nm;

FIGURE 7 is a graphical representation showing correlations between the degree of recovery of a spectral transmittance factor at a wavelength of 450 nm and visible light radiation time;

FIGURE 8 is a graphical representation showing correlations between the degree of recovery of spectral transmittance factor and visible light radiation time in different quantities of radiation;

FIGURE 9 is a graphical representation showing correlations between the degree of recovery of spectral transmittance factor and quantity of visible light radiation time; and

FIGURE 10 is a schematic illustration of an apparatus embodying the present invention.

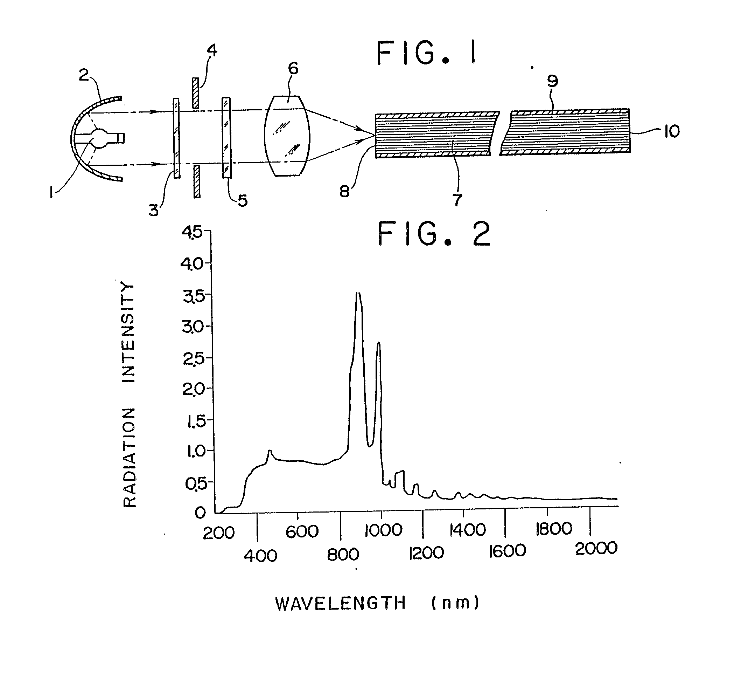

Referring now in detail to drawings, the apparatus illustrated in Figure 1 was used to carry out experimental measurements of correlations between wavelength and irradiation in fading of color of an image transmitting fiber bundle of a fiberoptic endoscope induced by irradiation. In Figure 1, light emitted backward from a xenon lamp 1 is reflected by a parabolic mirror 2 to be directed forward and the remainder which is emitted forward from a xenon lamp 1 falls directly upon a heat absorbing filter 3. Light passed through the heat absorbing filter 3 can fall upon a condenser lens or condenser lens assembly 6 through a stop 4 with a aperture of 28 mm and an interference filter 5. The condenser lens assembly 6 serves to use light emitted from the xenon lamp 1 effectively and to converge them onto an end surface 8 of an optical filter bundle 7 comprising an extremely large number of otical fibers each of diameter about 10 to 20 um. The optical fiber bundle 7 to be measured is identical with one used for an image transmitting fiber bundle of fiberoptic endoscope in every respect. That is to say, both ends of the optical fiber bundle 7 are in the form of a square with one side of 2 mm and have an overall length of 1250 mm. At each end, over a length of about 10 mm optical fibers of the fiber bundle 7 are rigidly maintained by an adhesive such as an epoxy resin so as to be exactly held in the same spatial relationship, and they are free along their path between the end faces so as to be flexible or freely movable. Furthermore the fiber bundle 7 is covered by a rubber tube for protection.

The fiber bundle 7 has been exposed, from the exit end to about 300 mm away therefrom, to 90R irradiation by a X-ray tube using 85 KVp and 300 mA, and thus colored yellow.

Figure 2 shows the radiation density distribution of the xenon lamp. The xenon lamp 1 has a density characteristic with flatness in the range of visible wavelength 380 to 780 nm.

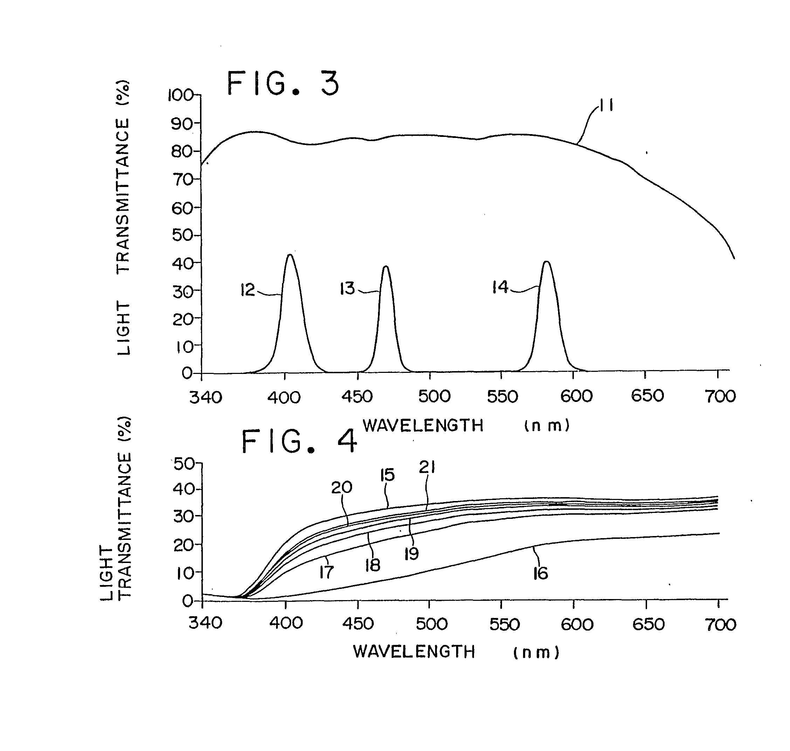

Figure 3 shows the spectral transmittance factors of a heat absorbing filter and an interference filter. The heat absorbing filter 3 which has a spectral transmittance factor represented by curve 11 can remove heat rays from radiation emitted from the xenon lamp 1. The heat absorbing filter 3 serves to prevent the epoxy resins used as adhesive for rigidly holding the fibers at their end portions from blackening which is caused upon heating of the epoxy resin higher than its heat-resisting temperature of about 80 to 100 degrees C. Differences in spectral transmittance factors of the fiber bundle 7 experimentally obtained after visible light radiation through interference filter 5 used selectively are represented by curves shown in Figures 4 to 7, said filters having their central wavelengths at 404 nm, 470 nm and 582 nm as shown by characteristic curves 12, 13 and 14 in Figure 3, respectively.

Figure 4 shows differences in the spectral transmittance factor of the fiber bundle 7 experimentally obtained after visible light irradiation at various periods of time through the interference filter having its central wavelength at 404 nm in the apparatus shown in Figure 1. The curve 15 in Figure 4 represents the spectral transmittance factor of the fiber bundle 7 before X-ray irradiation, and the curve 16 represents it after the fiber bundle 7 in the region from the light exit end 10 to about 300 mm away therefrom irradiated by X-rays of 90R and colored yellow. The fiber bundle 7 having its spectral transmittance factor lowered to that shown by the curve 16 was, at its end 8, radiated by visible light through the interference filter having its central wavelength at 404 for a quarter of an hour, for a half of an hour, for an hour, for two hours and for four hours. The recovery of spectral transmittance factor of the fiber bundle 7 represented by curves 17 to 21, respectively, was observed.

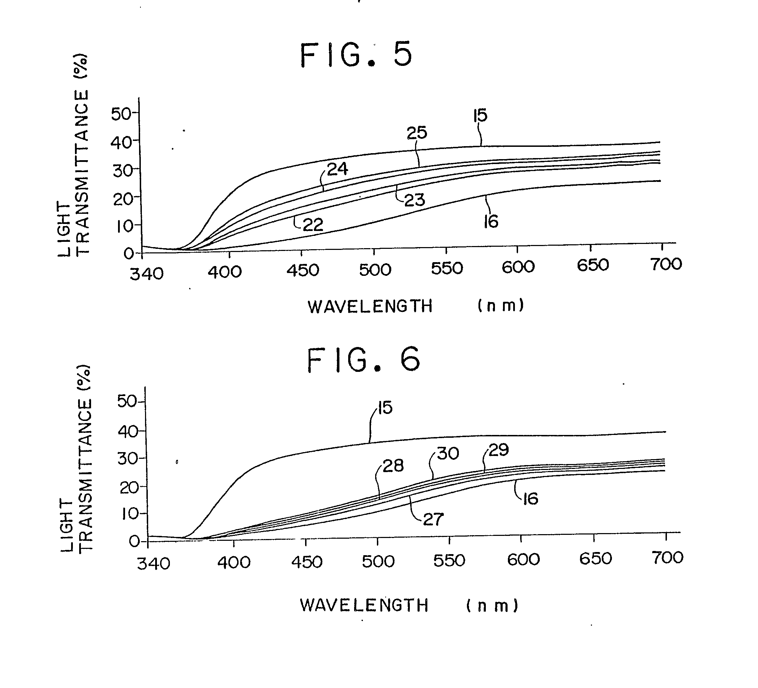

Figure 5 shows differences in spectral transmittance factor of the fiber bundle 7 experimentally obtained after visible light radiation for various periods of time through the interference filter having its central wavelength at 470 nm. The curves 22 to 25 represent the spectral transmittance factors of the fiber bundle 7 positively observed after visible light radiation of a quarter of an hour, for a half of an hour, for an hour and two hours, respectively. The curves 15 and 16 represent the spectral transmittance factor of the fiber bundle before and after X-ray irradiation, respecectively, in the same manner as shown by the curves in Figure 4.

Figure 6 shows differences in spectral transmittance factors of the fiber bundle 7 experimentally obtained after visible light radiation through the interference filter having its central wavelength at 582 nm. The curves 27 to 30 represent spectral transmittance factors of the fiber bundle recovered after visible light radiation for a quarter of an hour, for a half of an hour, for an hour and for two hours, respectively.

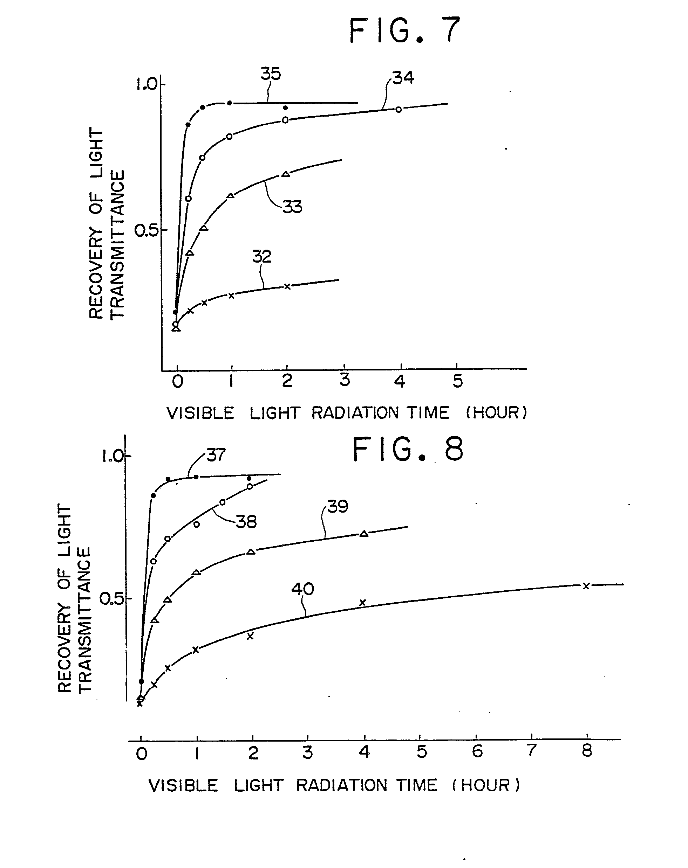

Figure 7 shows the recovery of light transmittance of the fiber bundle 7 with time by plotting the experimental values at the wavelength of 435 nm. Curves 32 to 34 showing the recovery of light transmittance at the wavelength of 435 nm of the fiber bundle 7 are in the case of using the interference filters having central wavelengths at 582 nm, 470 nm and 404, respectively, and curve 35 is without any interference filter. Here the recovery of light transmittance plotted along the ordinate represents the rate of light transmittance recovery to that before X-ray irradiation. As a result of investigating many data shown in Figure 7, there is every probability that visible light radiation of about 650 nm in wavelength will have the effect of causing the fiber bundle 7 to recover its light transmittance and that the shorter the wavelength of visible light radiation used is, the higher the recovery of light transmittance of the fiber bundle 7 in rate is. Further it will be apparant from Figure 7 that the rate of recovery of light transmittance of the fiber bundle 7 grows large at the range of about 500 to 470 nm in wavelength and extremely large at the range of about 400 to. 380 nm in wavelength. Consequently it is desirable to apply visible light radiation containing short wavelength components for the most efficient recovery of light transmittance of fiber bundles. On the other hand, the higher recovery in light transmittance will be obtained without using any interference filter. It is estimated as decribed hereinafter that this is due to visible light radiation which is higher in density and contains short wavelength components in the range of 380 to 400 nm.

An experiment for investigating the dependence of recovery of light transmittance on radiation density was made by the apparatus shown in Figure 1 by using some neutral density (ND) filters instead of the interference filter 5. Figure 8 shows relationships between the recovery of light transmittance at 450 nm in wavelength and visible light radiation time which were experimentally obtained. In Figure 8, curve 37 represents the recovery of light transmittance of a fiber bundle after visible light radiation without any ND filter, curve 38 represents the recovery of light transmittance of a fiber bundle after visible light radiation using a quarter of the total quantity of radiation by using a ND filter having a density of 4 (four), a curve 39 represents the recovery of light transmittance of a fiber bundle after visible light radiation using one sixteenth of the total quantity of radiation by using two ND filters having a density of 4 (four), and curve 40 represents the recovery of light transmittance after radiation reduced to 1/128 of the total quantity of radiation using two ND filters having a density of 4 and a ND filter having a density of 8. It will be apparant from Figure 8 that the larger the quantity the visible light radiation is, the higher the recovery of light transmittance is.

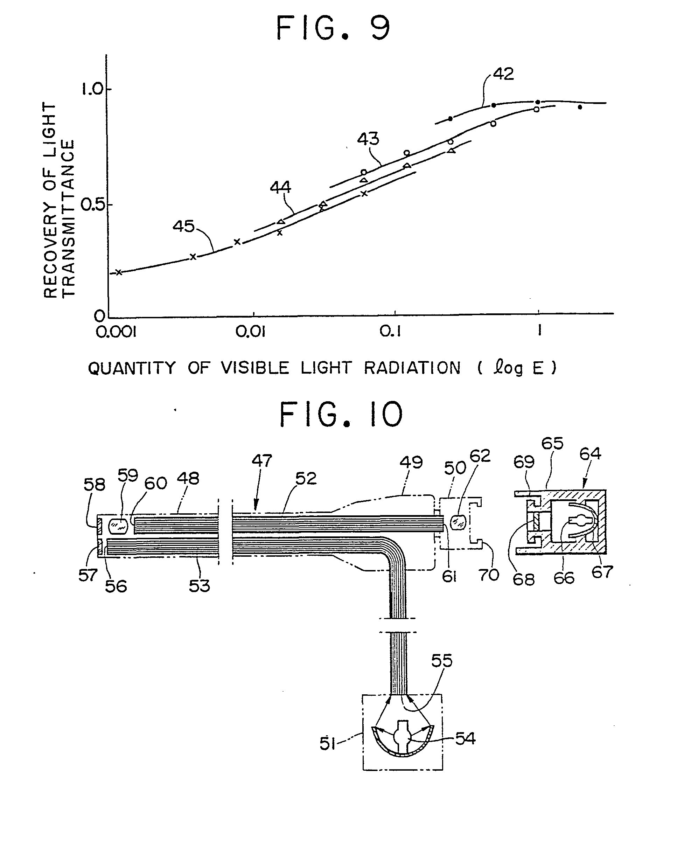

Figure 9 shows relationships between the recovery of light transmittance of a fiber bundle and the quantity of visible light radiation (log. E). In Figure 9, a characteristic curve 42 represents the recovery of light transmittance of a fiber bundle after visible light radiation without any filter, the characteristic curve 43 represents the recovery of light transmittance of a fiber bundle after visible radiation reduced to one quarter of the total quantity of radiation by using a filter, the characteristic curve 44 represents the recovery of transmittance after the radiation was reduced to one sixteenth, and the characteristic curve 45 represents the recovery of light transmittance after the radiation was reduced to 1/128th. It will be apparent from Figure 9 that the recovery of light transmittance in rate is not proportional to the quantity of visible light radiation and that the larger the quantity of visible light radiation is, the higher the recovery of light transmittance is.

As a result of synthesizing the whole experimental data strong visible light and short wavelength visible light can effectively recover the light transmittance of a fiber bundle which has gone yellow.

Figure 10 illustrates an embodiment of apparatus for restoring light transmittance of a fiber bundle within a fiberoptic endoscope. As is well known, a fiberoptic endoscope 47 comprises an elongated and flexible part 48 which is insertable into an body to be examined, a remote control part 49, an ocular part 50 and an illumination light source apparatus 51 and includes an image transmitting optical fiber bundle 52 and an illumination light transmitting optical fiber bundle 53 therein. The elongated and flexible part 48 is, as is well known, adapted to face the top thereof towards any desired direction by operating a control knob on the remote control part 49.

Light emitted from a light source 54 provided in the illumination light source apparatus 51 is incident into the light transmitting optical fiber bundle 53 through its incident end 55 and is emitted through the exit end 56. Illumination light can further emit through a window 57 to then illuminate the area to be examined or observed.

Reflected light from the area is converged onto an incident end 60 of the image transmitting optical fiber bundle 52 through the window 58 and an objective assembly 59. The image at exit end 61 of the image transmitting optical fiber bundle can be observed through an ocular assembly 62 after magnification.

When the fiberoptic endoscope 47 is used with a fluoroscopy employing a X-ray monitoring TV, the actual dose received by the fiber bundle within the flexible part 48 is about 0.1R per one examination. The light transmittance of the image transmitting optical fiber bundle is gradually reduced with yellowing thereof and consequently the image transmitting optical fiber bundle becomes unacceptable for use after a total dose of about 24R. Therefore it is desirable to restore the reduced light transmittance of the image transmitting optical fiber bundle after a certain cumulative dose for instance a total dose of about 1.0R by using the light transmittance restoring apparatus 64.

The light transmittance recovering apparatus 64 which comprises a light source 66 emitting radiation containing short wavelength visible light, a reflecting mirror 67 and a heat absorbing filter 68 in casing 65 is adapted to be detachably connected to the ocular assembly part 50. The connecting means is comprised as a bayonet mount with which a casing 65 is provided, The bayonet mount has bayonet detents 69 engageable with complementary bayonet detents 70 of the ocular assembly part 50 which are provided for mounting a camera or the like thereon.

A xenon lamp, a halogen lamp, a metal halogen lamp or the like may be employed as the light source 66. On the other hand, the illumination light source apparatus 51 can be used as a light transmittance restoring apparatus and in this case the illumination light source apparatus 51 is connected to the ocular assembly part 50, for example through a mounting means.

## Claims

1. A method of restoring the light transmittance of the image transmitting optical fiber bundle having an irradiation-induced reduction in light transmittance which method is characterised in that, the light transmitting bundle being the image transmitting bundle (52) of a fibre optic endoscope, and having an objective lens (50) at its distal end, said image transmitting optical fiber bundle is exposed to visible light radiation by directing said visible light radiation into the bundle through the end (61) from which said image is normally viewed.

2. A method as defined in claim 1, wherein said visible light radiation contains components of wavelength shorter than about 650 nm.

3. A method as defined in claim 1 or 2, wherein said visible light radiation contains components of wavelengths shorter than about 580 nm.

4. A method as defined in claim 1, 2 or 3, wherein said visible light radiation contains components of wavelength shorter than about 470 nm.

5. A method as defined in any one of claims 1 to 4, wherein said visible light radiation contains components of wavelength shorter than about 450 nm.

6. A method as defined in claim 4, wherein said visible light radiation contains components of wavelength shorter than about 400 nm.

7. A fibre optic endoscope comprising an image transmitting optical fiber bundle (52) having a light transmittance which in use is reduced by irradiation, the bundle having an end (61) from which a transmitted image is normally viewed and an objective lens assembly (59) at its distal end, characterised by the provision, in operative combination with the endoscope, of apparatus (64) for restoring the light transmittance of the bundle, the apparatus comprising:—

a light source (66) which, in use, emits visible light radiation; and

a casing (65) including said light source therein, the casing being adapted to be coupled to said end (61) of the bundle to direct light through the bundle.

8. A combination as defined in claim 7, wherein said light source (66) is a xenon lamp.

9. A combination as defined in claim 7, wherein said light source (66) is a halogen lamp.

10. A combination as defined in any one of claims 7 to 9, wherein the casing is provided with bayonet detents.

11. A combination as defined in any one of claims 7 to 10 which further comprises a heat absorbing filter (68) for absorbing thermal radiation contained within energy radiation emitted from said light source.

12. A combination as defined in claim 11, which further comprises a mirror (67) for reflecting light emitted from said light source forwardly.

## Patentansprüche

1. Verfahren zur Wiederherstellung der Lichtdurchlässigkeit eines Bildleitbündels aus optischen Fasern, das eine durch Bestrahlung hervorgerufene Verminderung in der Lichtdurchlässigkeit aufweist, dadurch gekennzeichnet, daß das Lichtleitbündel aus optischen Fasern, das das Bildleitbündel (52) eines faseroptischen Endoskops ist und ein Objektiv (59) an seinem distalen Ende hat, einer sichtbaren Lichtstrahlung ausgesetzt wird, indem die sichtbare Lichtstrahlung in das Bündel durch das Ende (61), von dem das Bild normalerweise betrachtet wird, geleitet wird.

2. Verfahren nach Anspruch 1, dadurch gekennzeichnet, daß die sichtbare Lichtstrahlung Wellenlängenkomponenten, die kürzer als etwa 650 nm sind, enthält.

3. Verfahren nach Anspruch 1 oder 2, dadurch gekennzeichnet, daß die sichtbare Lichtstrahlung Wellenlängenkomponenten, die kürzer als etwa 580 nm sind, enthält.

4. Verfahren nach Anspruch 1, 2 oder 3, dadurch gekennzeichnet, daß die sichtbare Lichtstrahlung Wellenlängenkomponenten, die kürzer als etwa 470 nm sind, enthält.

5. Verfahren nach einem der Ansprüche 1 bis 4, dadurch gekennzeichnet, daß die sichtbare Lichtstrahlung Wellenlängenkomponenten, die kürzer als etwa 450 nm sind, enthält.

6. Verfahren nach Anspruch 4, dadurch gekennzeichnet, daß die sichtbare Lichtstrahlung Wellenlängenkomponenten, die kürzer als etwa 400 nm sind, enthält.

7. Faseroptisches Endoskop mit einem Bildleitbündel (52) aus optischen Fasern, die eine im Gebrauch durch Bestrahlung verminderte Lichtdurchlässigkeit aufweisen, wobei das Bündel ein Ende (61), von dem aus normalerweise ein übertragenes Bild betrachtet wird, und an seinem distalen Ende eine Objektivsystem (59) hat, gekennzeichnet durch eine mit dem Endoskop in Wirkverbindung zu bringende, die Lichtdurchlässigkeit des Bündels wiederherstellende Vorrichtung (64), die
— eine Lichtquelle (66), die im Betrieb eine sichtbare Lichtstrahlung aussendet, und
— ein die Lichtquelle in seinem Inneren aufnehmendes Gehäuse (65), das mit dem Ende (61) des Bündels, um Licht durch das Bündel zu leiten, verbindbar ist, aufweist.

8. Anordnung nach Anspruch 7, dadurch gekennzeichnet, daß die Lichtquelle (66) eine Xenonlampe ist.

9. Anordnung nach Anspruch 7, dadurch gekennzeichnet, daß die Lichtquelle (66) eine Halogenlampe ist.

10. Anordnung nach einem der Ansprüche 7 bis 9, dadurch gekennzeichnet, daß das Gehäuse mit Bajonettriegeln versehen ist.

11. Anordnung nach einem der Ansprüche 7 bis 10, gekennzeichnet durch ein Wärmestrahlung, die in der von der Lichtquelle ausgesandten Energiestrahlung enthalten ist, absorbierendes Wärmeschutzfilter (68).

12. Anordnung nach Anspruch 11, gekennzeichnet durch einen von der Lichtquelle ausgesandtes Licht vorwärts reflektierenden Spiegel (67).

## Revendications

1. Procédé pour rétablir le facteur de transmission de la lumière du faisceau de fibres optiques transmettant une image présentant une réduction de son facteur de transmission de la lumière provoquée par une exposition à un rayonnement, caractérisé en ce que, le faisceau transmettant la lumière étant le faisceau transmettant une image (52) d'un endoscope à optique en fibres et présentant un montage de lentilles formant un objectif (59) à son extrémité distale, on expose ce faisceau de fibres optiques transmettant une image à un rayonnement lumineux visible en dirigeant le rayonnement lumineux visible dans le faisceau à travers l'extrémité (61) de laquelle l'image est normalement observée.

2. Procédé suivant la revendication 1, dans lequel le rayonnement lumineaux visible contient des composantes de longueurs d'ondes inférieures à environ 650 nm.

3. Procédé suivant la revendication 1 ou 2, dans lequel le rayonnement lumineux visible contient des composantes de longueurs d'ondes inférieures à environ 580 nm.

4. Procédé suivant la revendication 1, 2 ou 3, dans lequel le rayonnement lumineux visible contient des composantes de longueurs d'ondes inférieures à environ 470 nm.

5. Procédé suivant l'une quelconque des revendications 1 à 4, dans lequel le rayonnement lumineux visible contient des composantes de longueurs d'ondes inférieures à environ 450 nm.

6. Procédé suivant la revendication 4, dans lequel le rayonnement lumineux visible contient des composantes de longueurs d'ondes inférieures à environ 400 nm.

7. Endoscope à optique en fibres comprenant un faisceau de fibres optiques transmettant une image (52) ayant un facteur de transmission de la lumière qui, en service, est réduit par exposition à un rayonnement, le faisceau comportant une extrémite (61) à partir de laquelle une image transmise est normalement observée et un montage de lentilles formant un objectif (59) à son extrémité distale, caractérisé en ce qu'en combinaison avec l'endoscope, il est prévu un appareil (64) pour rétablir le facteur de transmission de la lumière du faisceau, cet appareil comprenant:

une source de lumière (66) qui, en service, émet un rayonnement lumineaux visible, et

un boîtier (65) contenant la source de lumière, le boîtier étant destiné à être raccordé à l'extrémité (61) du faisceau pour diriger de la lumière à travers le faisceau.

8. Combinaison suivant la revendication 7, dans laquelle la source de lumière (66) est une lampe au xénon.

9. Combinaison suivant la revendication 7, dans laquelle la source de lumière (66) est une lampe à halogène.

10. Combinaison suivant l'une quelconque des revendications 7 à 9, dans laquelle le boîtier est pourvu de dents de baïonnette.

11. Combinaison suivant l'une quelconque des revendications 7 à 10, qui comprend, en outre, un filtre absorbant la chaleur (68) pour absorber le rayonnement thermique contenu dans un rayonnement énergétique émis par la source de lumière.

12. Combinaison suivant la revendication 11, qui comprend, en outre, un miroir (67) pour réfléchir vers l'avant la lumière émise par la source de lumière.

FIG. 1

FIG. 2

RADIATION INTENSITY

WAVELENGTH (nm)

FIG. 3

LIGHT TRANSMITTANCE (%)

WAVELENGTH (nm)

FIG. 4

LIGHT TRANSMITTANCE (%)

WAVELENGTH (nm)

FIG. 5

FIG. 6

0 058 574

0 058 574

FIG. 7

FIG. 8

4

# FIG. 9

# FIG. 10